(19) Europäisches Patentamt
European Patent Office
Office européen des brevets

(11) Publication number: **0 064 781**
**B1**

(12) **EUROPEAN PATENT SPECIFICATION**

(45) Date of publication of patent specification: **23.05.84**

(51) Int. Cl.³: **C 07 C121/46**, C 07 C 61/40

(21) Application number: **82200456.0**

(22) Date of filing: **14.04.82**

(54) **Process for the preparation of cyclopropane compounds.**

(30) Priority: **30.04.81 GB 8113359**

(43) Date of publication of application:
**17.11.82 Bulletin 82/46**

(45) Publication of the grant of the patent:
**23.05.84 Bulletin 84/21**

(84) Designated contracting states:
**BE CH DE FR GB IT LI NL**

(56) References cited:
**EP-A- 0 003 683**
**BE-A- 704 983**
**DE-A- 2 621 830**
**DE-A- 2 727 613**
**US-A- 4 174 348**
**ANGEWANDTE CHEMIE, (International Edition), Vol. 20, No. 9, September 1981, Weinheim D. ARLT et al. "Syntheses of Pyrethroid Acids", pages 703 to 722**
**JOURNAL OF ORGANIC CHEMISTRY, Vol. 44, No. 3, February 1979, Easton P.D. KLEMMENSEN et al. "Convenient Synthesis of Ethyl (+, −)-cis- and trans-3-(2,2Dichloroethenyl)-2,2-dimethyl-cyclopropanecarboxylate", pages 416 to 420**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **de Nie-Sarink, Margaretha Johanna**
**Badhuisweg 3**
**NL-1031 CM Amsterdam (NL)**
Inventor: **van Helden, Robert**
**Badhuisweg 3**
**NL-1031 CM Amsterdam (NL)**
Inventor: **Schmitz, Felix**
**Carel van Bylandtlaan 30**
**NL-2596 HR The Hague (NL)**

(74) Representative: **Hunter, Keith Roger Ian et al**
**4 York Road**
**London SE1 7NA (GB)**

**EP 0 064 781 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

This invention relates to a process for the preparation of cyclopropane compounds.

Synthetic pyrethroid insecticides are esters which consist of an acid portion and an alcohol portion. In one group of pyrethroids, the acid portion is derived from a 2,2-dihalovinylcyclopropane carboxylic acid, and a great deal of research has been directed towards the preparation of these acids.

Many research programmes have been directed towards a method of formation of the cyclopropane ring by base-catalysed cyclization of suitably substituted compounds related to hexanoic acid. In the most general of terms, such reactions can be represented as follows :

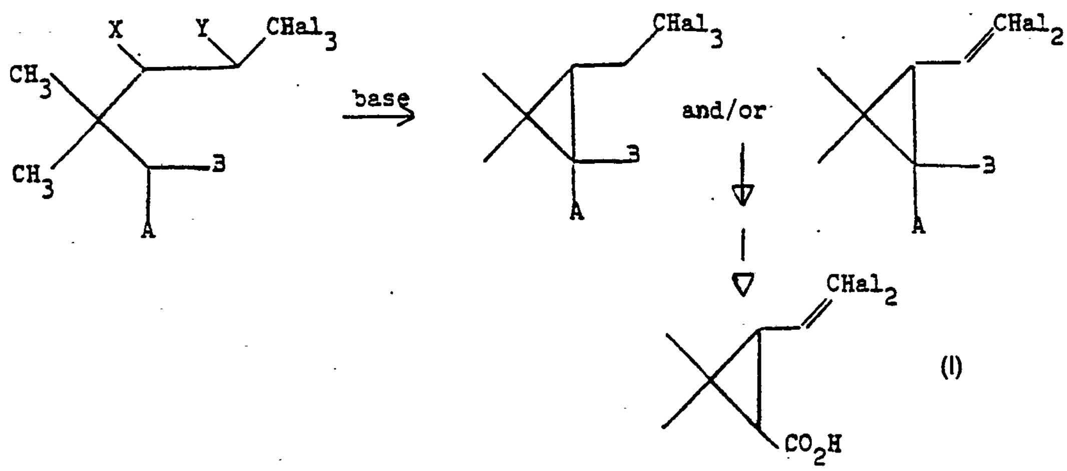


(I)

Here Hal is halogen and either X is halogen and Y is hydrogen or X and Y together form an olefinic double bond. In the prior art, A and B can represent a variety of possible groups such that the desired acid (or an ester thereof) can be prepared. Examples of documents in which such reactions are disclosed are German Offenlegungsschrift 2606635 und UK Specifications 1561502, 1512419, 1520023 and 1580203.

In general these reactions proceed smoothly and in good yield. However, as will be appreciated, the desired end-product (I) can exist as two geometric isomers, in which the $-CH=CHal_2$ and $-CO_2H$ groups are cis or trans to each other. (Each of these geometric isomers, of course, exists as two optical isomers.) Synthetic pyrethroids in which the acid portion is in the cis form are in general more active as insecticides than the corresponding trans compound. One of the problems associated with the above type of reaction is that it tends in general to produce a mixture of the two possible products. Often the ratio of the two products is strongly dependent on the reaction conditions. Again, much research has been directed towards the development of a process which will produce the desired cis acid in large excess over the trans acid. European Patent Application N° 3683 describes one such process, in which the compound cyclised is an ester, and cyclisation occurs in such a way that the halogen-containing group and the ester group are cis to each other.

The Applicants have now found that when using a specific type of hexanoic acid derivative as starting material, a specific type of base and a specific type of reaction medium, cyclisation can occur to give a compound containing a carboxyl group and a halogen-containing group trans to each other. This intermediate compound can be readily converted into the desired cis acid or ester.

The invention therefore provides a process for the preparation of a high trans compound (as hereinafter defined) of the general formula

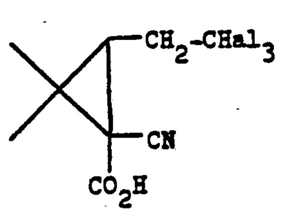 (II) and/or 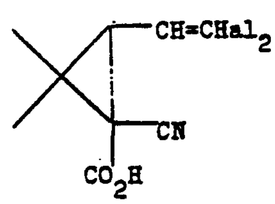 (III)

or an alkali metal salt thereof, in which each Hal independently represents a chlorine or bromine atom, which comprises reacting a compound of the general formula

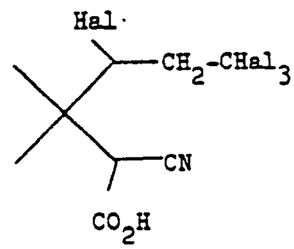

(IV)

or an alkali metal salt thereof, in which each Hal independently represents a chlorine or bromine atom, with an alkali metal hydroxide, the reaction taking place in solution or suspension in an organic liquid phase having a dielectric constant of less than 10.

Preferably each Hal in the formula IV represents the same type of halogen atom, especially a chlorine atom.

The term trans applied to the compounds of formula II and III means the isomer in which the $—CH_2—CHal_3$ or $—CH = CHal_2$ group and the $—CO_2H$ group are trans to each other, thus :

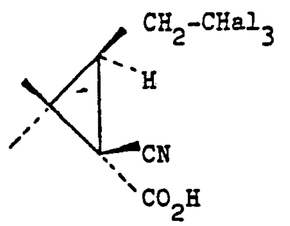

(II) trans
(2 possible optical isomers)

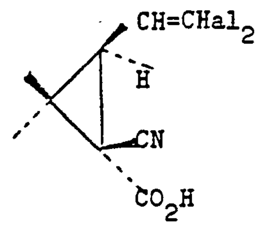

(III) trans
(2 possible optical isomers)

and the term " cis " should be construed accordingly. The term " high trans " applied to the compounds II and III should be understood to mean-that the trans isomer is present in an amount of at least 60 % of total compound i. e. that the trans : cis ratio is at least 60 : 40. In practice, it is possible by using the process according to the invention under optimum conditions to obtain trans : cis ratios much higher than 60 : 40.

The organic liquid phase having a dielectric constant of less than 10, may comprise a single organic compound or a mixture of two or more organic compounds. The dielectric constants of liquids show a small temperature dependence and the liquid should be understood to have a dielectric constant of less than 10 measured at 20 °C or, if the melting point is higher than 20 °C, at just above the melting point. Dielectric constants of most common organic solvents can be found for example in Techniques of Chemistry vol. II, " Organic Solvents " by J. A. Riddick and W. B. Bunger, or in the " Handbook of Chemistry and Physics " published by the Chemical Rubber Publishing Co. Ltd.

Suitable organic liquids may for example be selected from the following general groups of compounds : for each specific compound the figure in brackets is the dielectric constant : hydrocarbons and chlorinated hydrocarbons, for example benzene (2.3), toluene (2.4), chlorobenzene, hexane, cyclohexane, dichloromethane and carbon tetrachloride ; ethers such as diethyl ether and dioxane ; esters such as ethyl acetate ; and alcohols such as menthol and t-amyl alcohol. The only essential feature of the liquid is that it should have the required dielectric constant. In general, the trans : cis ratio of the products II and/or III tends to increase with decreasing dielectric constant. Thus the dielectric constant is preferably less than 5, especially less than 4.

The starting material IV (or its alkali metal salt) may be fully soluble in the organic liquid phase having a dielectric constant of less than 10 but this is not essential. The reaction will also proceed to give a high trans product with solid starting material IV in suspension in the organic phase : in such a case, it is possible that the reaction takes place at the interface between the solid and the organic liquid, or that the reaction occurs via small amounts of starting material in solution. In some cases, it has been found desirable to include in the organic phase, a liquid with a very low dielectric constant, such as carbon tetrachloride, in which the starting material is very poorly soluble, together with a liquid with a higher dielectric constant in order to increase the solubility of the starting material. This may increase the rate of the reaction and/or the handling properties of the reaction mixture. The overall dielectric constant of this mixture should not of course exceed 10.

While it is not essential that the starting material be soluble in the organic liquid phase having a dielectric constant of less than 10, steps should be taken to ensure that the starting material does not dissolve to any great extent in any other liquid phase present which does not have a dielectric constant of less than 10. Thus for example if it is desired to dose the hydroxide to the reaction mixture in the form of an aqueous solution, this is possible provided that steps are taken to ensure that the starting material does not dissolve to any significant extent in the aqueous phase. This may be done by ensuring that the

aqueous phase contains a sufficiently high concentration of base that the starting material cannot dissolve in significant amounts therein. If however dilute solutions of hydroxide are dosed to the reaction mixture, the starting material will dissolve in the water, and reaction will take place in that phase reducing the trans : cis ratio.

Sodium, potassium and lithium hydroxides are all suitable for use in the process according to the invention, with lithium hydroxide usually giving marginally higher trans : cis ratios in the compounds II and/or III. The base may be added to the reaction mixture in solid form. If it is desired to use the base in the form of an aqueous solution, this is possible subject to the constraints of solubility of the starting material discussed above. In general, in order to prevent the dissolution of large quantities of the starting material in the aqueous phase, any aqueous hydroxide added preferably contains at least 20 % by weight of hydroxide. Such a high concentration is not possible with lithium hydroxide, which is not very soluble in water, so lithium hydroxide is most conveniently dosed as solid LiOH or $LiOH \cdot H_2O$.

The stoichiometry of the ring closure reaction to an alkali metal salt of compound II requires the use of two moles of hydroxide per mole of starting material when starting from the free acid IV, and one mole per mole when starting from an alkali metal salt of the acid. In general, the use of rather higher quantities of hydroxide is preferred ; for example an excess of at least 0.25 moles hydroxide per mole of starting material, preferably an excess of from 0.25 to 5 moles per mole, may be used. Reaction times may of course vary enormously depending on the precise reaction conditions, but times of less than 8 hours, often around 4 hours, five generally acceptable results. The immediate product of the reaction is normally an alkali metal salt of the compound II and/or III, which can of course be converted to the free acid if desired.

The process according to the invention is preferably conducted at a temperature in the range of from 0 to 100 °C, especially 10 to 50 °C. As discussed below, relatively high temperatures, for example greater than 60 °C, tend to promote the formation of greater quantities of compounds of formula III relative to compounds of formula II.

Whether a compound of formula II or of formula III (or an alkali metal salt thereof) is obtained in the process according to the invention when using excess hydroxide, depends on the precise reaction conditions.

In general, the base-induced cyclisation to the compound of formula II takes place at a lower temperature than that required to dehydrohalogenate a $—CH_2—CHal_3$ group to a $—CH = CHal_2$ group. Thus by maintaining a relatively low reaction temperature, it is possible to obtain a product containing compound II together with very little or no compound III. Under most circumstances, this is the more desirable way to conduct the reaction, since it has been found that at the higher temperatures required to produce high yields of compound III directly in the process of the invention, an undesired side reaction occurs : further dehydrohalogenation to produce a haloacetylene group, $—C \equiv CHal$, occurs. Furthermore, this reaction appears to proceed more readily with the trans isomer than with the cis isomer, thus reducing not only the chemical yield but also the trans : cis ratio of the product. It has further been found that the dehydrohalogenation to the acetylene occurs more readily in solvents of lower dielectric constant than in solvents of higher dielectric constant. Thus a preferred mode of operation is to conduct the reaction according to the invention under conditions favouring formation of the compound of the general formula II, and then subsequently to increase the dielectric constant of the reaction medium, either by completely replacing the solvent or by adding a high dielectric constant solvent, such as methanol, and to raise the temperature to produce the compound of the general formula III. The dehydrohalogenation reaction, of course, consumes one additional molar equivalent of a base, which may be an alkali metal hydroxide or a different base, as desired.

The accompanying drawing shows schematically a series of reactions for the preparation of the desired dihalovinyl acid I from a compound of the general formula IV. For clarity, all compounds have been shown as free acids rather than their alkali metal salts, and only the reactions of the desired isomers have been shown. Thus a resulting compound II may be dehydrohalogenated and decarboxylated, in one or two steps, and a resulting compound III may be decarboxylated, to give the cyano compound V. This compound may be hydrolysed by known methods to give the desired acid I. Alternatively the cyano compound V may be alcoholized by known methods to give an ester of the acid I.

The starting material IV may be prepared by any suitable method, for example by reaction of the compound :

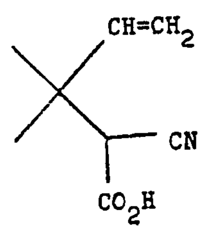

with a carbon tetrahalide, $CHal_4$, in the presence of a catalyst, for example an iron salt plus benzoin. If this

reaction is carried out in the presence of a suitable solvent system for example in excess $CHal_4$ optionally together with a suitable co-solvent, the resulting reaction mixture containing the compound IV may if desired be reacted directly in the process according to the present invention without intermediate work-up.

The following Examples illustrate the invention. Throughout the Examples, 2,2-dimethyl-3-(2,2,2-trichloroethyl)-1-cyanocyclopropane carboxylic acid is referred to as A, and 2,2-dimethyl-3-(2,2-dichloroethenyl)-1-cyanocyclopropane carboxylic acid is referred to as B. Analyses were carried out using NMR and/or gas-liquid chromatography.

## Example 1

Potassium hydroxide (0.5 g, 8,93 mmol) was added in solid form to a vigorously stirred suspension of 4,6,6,6-tetrachloro-3,3-dimethyl-2-cyanohexanoic acid (0.5 g, 1.63 mmol) in 5 ml of toluene. The reaction mixture was stirred for 4 hours at room temperature. The reaction mixture was taken up in water, acidified with HCl and thoroughly extracted with diethylether. The combined ethereal extracts were dried over anhydrous magnesium sulphate. Evaporation of the solvent in vacuo gave a solid product. Analysis of the product by NMR showed the following distribution :

cis/trans-2,2-dimethyl-3-(2,2,2-trichloroethyl)-1-cyanocyclopropane carboxylic acid (A)
cis/trans-2,2-dimethyl-3-(2,2-dichloroethenyl)-1-cyanocyclopropane carboxylic acid (B)
A : B = 93 : 7, cis : trans ration 14 : 86.

## Example 2

The product from Example 1 was dissolved in 5 ml of methanol containing potassium hydroxide (0.5 g, 8.93 mmol) and stirred for 4 hours at reflux temperature. The reaction mixture was allowed to cool to room temperature, taken up in water, acidified with HCl and thoroughly extracted with diethylether. The combined ethereal extracts were dried over anhydrous magnesium sulphate. Evaporation of the solvent gave a solid product. Analysis of the product by NMR showed the following distribution :

cis-2,2-dimethyl-3-(2,2-dichloroethenyl)-1-cyanocyclopropane carboxylic acid (cis B)
trans-2,2-dimethyl-3-(2,2-dichloroethenyl)-1-cyanocyclopropane carboxylic acid (trans B)
cis : trans ratio = 14 : 86.

## Example 3

Potassium hydroxide (0.5 g, 8.93 mmol) was added in solid form to a vigorously stirred suspension of 4,6,6,6-tetrachloro-3,3-dimethyl-2-cyanohexanoic acid (0.5 g, 1.63 mmol) in 10 ml of toluene. The reaction mixture was stirred for 4 hours at room temperature. After addition of 5 ml of methanol the mixture was heated to reflux temperature and stirred for an additional 4 hours. The reaction mixture was allowed to cool to room temperature, taken up in water, acidified with HCl and thoroughly extracted with diethylether. The combined ethereal extracts were dried over anhydrous magnesium sulphate. Evaporation of the solvent gave solid B. Analysis of this product by NMR showed that it had a cis : trans ratio of 15 : 85.

## Examples 4 to 8

The procedure of Example 1 was followed except that the toluene was replaced by the stated organic liquids. In Example 8, the reaction was conducted at 80 °C, menthol being a solid at room temperature. In all cases, the main product obtained was A, with only small quantities of B present. The results are given in Table 1.

Table 1

| Example No. | Solvent | cis:trans ratio of product |
|---|---|---|
| 3 | toluene:dioxane 1:1 by volume | 14:86 |
| 4 | diethylether | 18:82 |
| 5 | dichloromethane | 23:77 |

Table 1 (Continued)

| Example No. | Solvent | cis:trans ratio of product |
|---|---|---|
| 6 | ethyl acetate | 22:78 |
| 7 | dioxane | 14:86 |
| 8 | menthol | 25:75 |

Examples 9 to 13

A series of experiments were carried out as follows. 1 g finely powdered 4,6,6,6-tetrachloro-3,3-dimethyl-2-cyanohexanoic acid was added to a mixture of 8 g carbon tetrachloride and 2 g methyl isobutyl ketone. The appropriate base was then added, and the reaction mixture was stirred at room temperature for the stated reaction time. In all cases, the main product was A, with only small quantities of B present. The results are given in Table 2.

Table 2

| Example no. | Hydroxide added | molar ratio base:starting material | reaction time (hours) | cis:trans ratios of products |
|---|---|---|---|---|
| 9 | KOH pellets | 2.4:1 | 4 | 15:85 |
| 10 | NaOH pellets | 2.4:1 | 4 | 12:88 |
| 11 | NaOH 30% w. aqueous solution | 2.6:1 | 1 | 16:84 |
| 12 | NaOH 55% w. aqueous solution | 3.0:1 | 1 | 12:88 |
| 13 | LiOH powder | 2.4:1 | 6 | 9:91 |

Example 14

Example 13 was repeated exactly except that the solvent was replaced by 5 g carbon tetrachloride and 0.33 g methyl isobutyl ketone. The cis : trans ratio of the product was 7 : 93.

Example 15

Example 12 was repeated exactly, except that the following additional components were added, expressed as moles per mole of 4,6,6,6-tetrachloro-3,3-dimethyl-2-cyanohexanoic acid : benzil (0.25), $FeCl_3$ (0.10) and LiCl (0.10). (This system models a reaction mixture in which the starting material has been generated *in situ* by iron-catalysed addition of carbon tetrachloride to 2-cyano-3,3-dimethyl-4-pentenoic acid.) The cis : trans ratio of the product was 16 : 84. When this experiment was repeated at a temperature of 50 °C instead of room temperature, the cis : trans ratio obtained was 14 : 86.

Examples 16 to 18 (comparison)

The procedure of Example 1 was followed except that the toluene was replaced by various solvents having a dielectric constant of greater then 10. The results are shown in Table 3 below.

Table 3

| Example No. | Solvent | Molar ratio A:B produced | cis:trans ratio of product |
|---|---|---|---|
| 16 | methanol | Not determined | 42:58 |
| 17 | acetonitrile | 77:23 | 45:55 |
| 18 | water | only trace B | 56:44 |

## Claims

1. A process for the preparation of a high trans compound (as hereinbefore defined) of the general formula

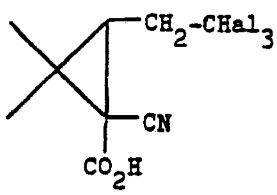 (II) and/or 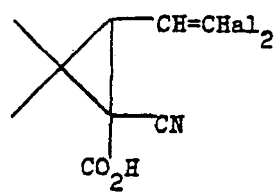 (III)

or an alkali metal salt thereof, in which each Hal independently represents a chlorine or bromine atom, which comprises reacting a compound of the general formula

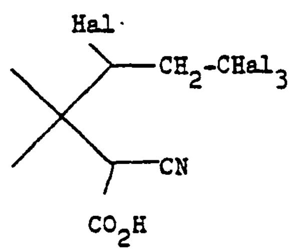 (IV)

or an alkali metal salt thereof, in which each Hal independently represents a chlorine or bromine atom, with an alkali metal hydroxide, the reaction taking place in solution or suspension in an organic liquid phase having a dielectric constant of less than 10.

2. A process as claimed in claim 1, in which each Hal in the general formula IV represents a chlorine atom.

3. A process as claimed in either claim 1 or claim 2, in which said organic liquid phase has a dielectric constant of less than 5.

4. A process as claimed in any one of claims 1 to 3, in which said organic liquid phase comprises one or more liquids selected from the following classes of compounds: hydrocarbons; chlorinated hydrocarbons; ethers; esters; and alcohols.

5. A process as claimed in any one of claims 1 to 4, carried out at a temperature in the range of from 0 to 100 °C.

6. A process as claimed in claim 5, carried out at a temperature in the range of from 10 to 50 °C.

7. A high trans compound of the general formula II or III as defined in claim 1, or an alkali metal salt thereof, whenever prepared by a process as claimed in any one of claims 1 to 6.

8. The use of a compound as claimed in claim 7 in a process for the preparation of an acid of the general formula

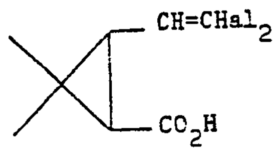

(V)

or a salt or an ester thereof, in which each Hal independently represents a chlorine or bromine atom.

**Ansprüche**

1. Verfahren zur Herstellung einer weitgehend trans-Verbindung (wie vorher definiert) der allgemeinen Formel

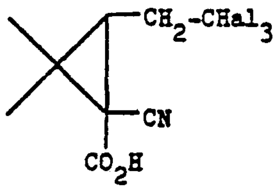

(II) und/oder

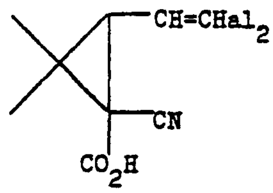

(III)

oder eines Alkalisalzes davon, wobei jedes Hal unabhängig ein Chlor- oder Bromatom bedeutet, umfassend die Umsetzung einer Verbindung der allgemeinen Formel

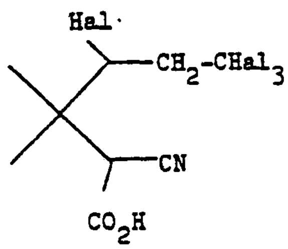

(IV)

oder eines Alkalisalzes davon, in der jedes Hal unabhängig ein Chlor- oder Bromatom bedeutet, mit einem Alkalihydroxid, wobei die Reaktion in Lösung oder Suspension in einer organischen flüssigen Phase mit einer Dielektrizitätskonstante von weniger als 10 abläuft.

2. Verfahren nach Anspruch 1, wobei jedes Hal bei der allgemeinen Formel IV ein Chloratom bedeutet.

3. Verfahren nach einem der Ansprüche 1 oder 2, wobei die organische flüssige Phase eine Dielektrizitätskonstante von weniger als 5 besitzt.

4. Verfahren nach einem der Ansprüche 1 bis 3, wobei die organische flüssige Phase, eine oder mehrere Flüssigkeiten, ausgewählt aus den folgenden Gruppen von Verbindungen, umfaßt: Kohlenwasserstoffe, chlorierte Kohlenwasserstoffe, Ether, Ester und Alkohole.

5. Verfahren nach einem der Ansprüche 1 bis 4, das bei einer Temperatur im Bereich von 0 bis 100 °C durchgeführt wird.

6. Verfahren nach Anspruch 5, wobei bei einer Temperatur im Bereich von 10 bis 50 °C gearbeitet wird.

7. Weitgehend trans.-Verbindung der allgemeinen Formel II oder III wie in Anspruch 1 angegeben oder ein Alkalisalz davon, hergestellt nach einem Verfahren wie in einem der Ansprüche 1 bis 6 beansprucht.

8. Verwendung einer Verbindung nach Anspruch 7 in einem Verfahren zur Herstellung einer Säure der allgemeinen Formel

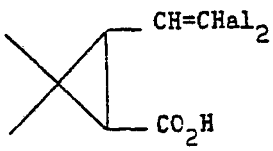

(V)

oder eines Salzes oder eines Esters davon, wobei jedes Hal unabhängig ein Chlor- oder Bromatom bedeutet.

**Revendications**

1. Un procédé pour la préparation d'un composé d'une haute teneur en trans (comme défini ci-dessus) de la formule générale

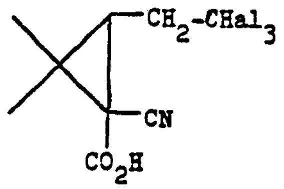 (II) et/ou 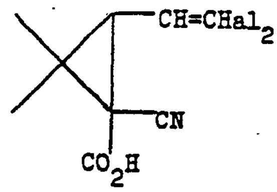 (III)

ou d'un sel de métal alcalin d'un tel composé, où chaque Hal indépendamment représente un atome de chlore ou de brome, selon lequel on fait réagir un composé de la formule générale

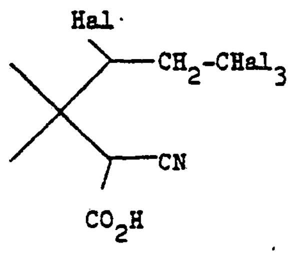 (IV)

ou un sel de métal alcalin d'un tel composé, où chaque Hal indépendamment représente un atome de chlore ou de brome, avec un hydroxyde de métal alcalin, la réaction ayant lieu en solution ou en suspension dans une phase liquide organique ayant une constante diélectrique de moins de 10.

2. Un procédé selon la revendication 1, dans lequel chaque Hal dans la formule générale IV représente un atome de chlore.

3. Un procédé selon la revendication 1 ou 2, dans lequel la phase liquide organique a une constante diélectrique de moins de 5.

4. Un procédé selon l'une quelconque des revendications 1 à 3, dans lequel la phase liquide organique comprend un ou plusieurs liquides choisis dans les classes suivantes de composés : hydrocarbures ; hydrocarbures chlorés ; éthers ; esters et alcools.

5. Un procédé selon l'une quelconque des revendications 1 à 4, mis en œuvre à une température comprise entre 0 et 100 °C.

6. Un procédé selon la revendication 5, mis en œuvre à une température comprise entre 10 et 50 °C.

7. Un composé d'une haute teneur en trans de la formule générale II ou III comme défini dans la revendication 1, ou un sel de métal alcalin d'un tel composé, chaque fois qu'il est préparé par un procédé selon l'une quelconque des revendications 1 à 6.

8. L'utilisation d'un composé selon la revendication 7 dans un procédé pour la préparation d'un acide de la formule générale

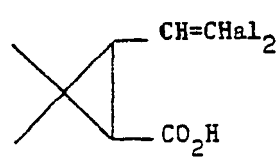 (V)

ou d'un sel ou ester d'un tel acide, où chaque Hal indépendamment représente un atome de chlore ou de brome.

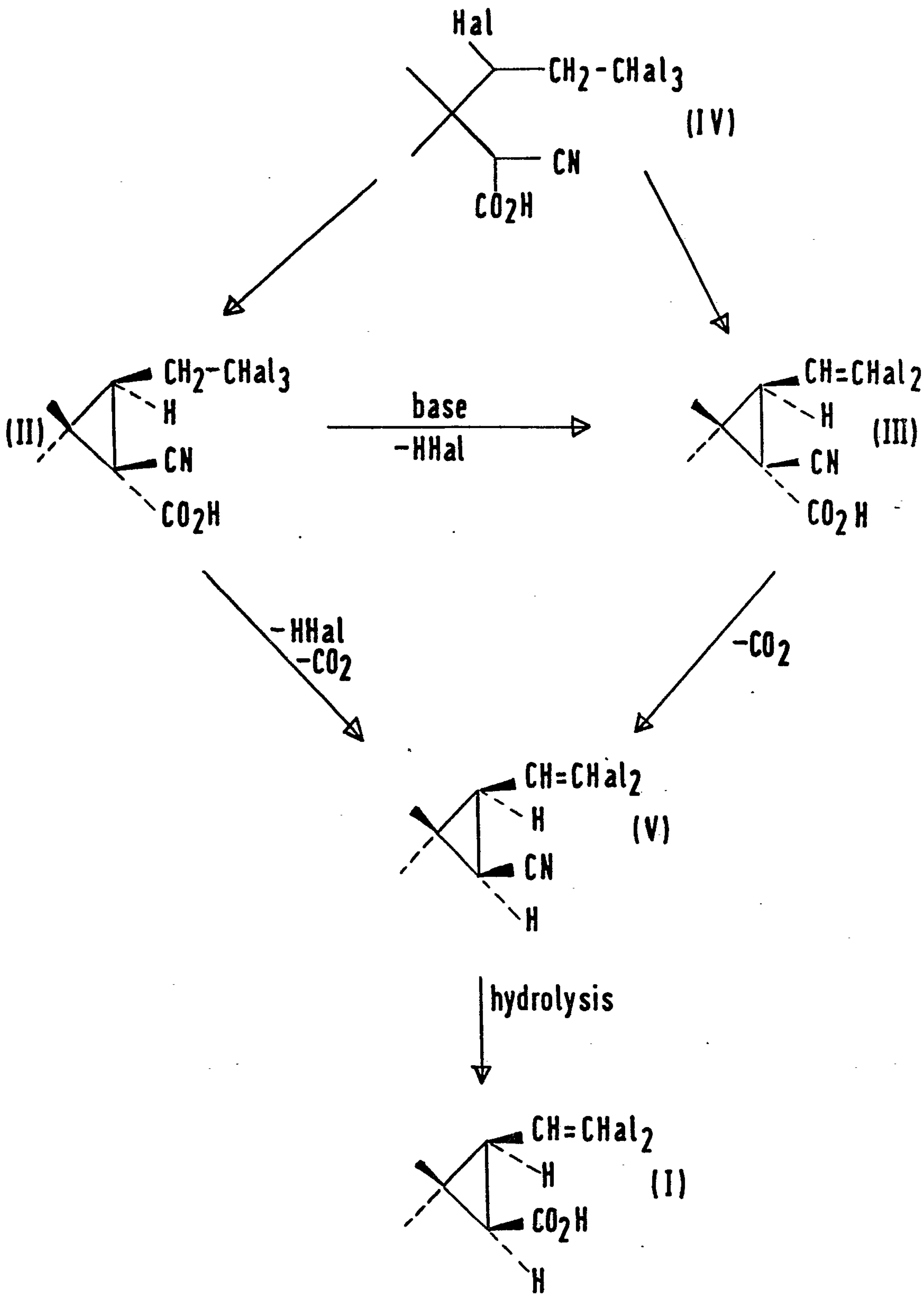
(IV)
(II)
base
-HHal
(III)
-HHal
$-CO_2$
$-CO_2$
(V)
hydrolysis
(I)